# EUROPEAN PATENT APPLICATION

(11) **EP 1 302 888 A2**
(43) Date of publication of application: **16.04.2003**
(21) Application number: 02256921.4
(22) Date of filing: 04.10.2002
(51) Int. Cl.: G06F 19/00

(54) **A system and method for use in providing a healthcare information database**

(30) Priority: 10.10.2001 US 328354 P; 16.09.2002 US 244443
(71) Applicant: Siemens Medical Solutions Health Services Corporation, Malvern, PA 19355 (US)
(72) Inventor: Anderson, Laura M., Downingtown, PA 19335 (US); Denny, Robert C., West Chester, PA 19380 (US)
(74) Representative: Wilding, Frances Ward

(57) **Abstract**

A method for providing accessibility to an information database by users of application software is presented, comprising the steps of retrieving desired data from a first source; processing the data to be suitable for incorporation in a transitional database (40) by using industry specific starter data (35) to aid in merging the desired data; applying a predetermined rule (22) to the processed data to ensure compatibility of the processed data with the transitional database (40); incorporating the processed data into the transitional database in response to the determination; and communicating transitional database data to a targeted information database (62).

## Description

This invention concerns a system and user interface for processing healthcare and patient related information to create a database supporting a healthcare information system for use by hospitals, or other healthcare delivery enterprises for example.

As a software application is used, data accumulate within its databases. As newer systems come into existence, inclusion of data from the older system is often desired or required by the new system. However, there are often great disparities between the existing data and the new data, e.g. in format, layout, and the like.

Solutions to importing data from an existing (or "legacy") system's database tables (i.e., its "legacy data") into a new system's database tables exist in the art, but no solution has addressed using an intermediary database that is at least partially populated with industry specific data and/or data definitions where the intermediary database is formatted for compatibility with the new system and capable of merging legacy data from the existing system in a predetermined manner.

Additionally, the prior art does not provide for transitioning from an existing application system and its data in such a manner as to enable a customer to initiate operation of a new system with a fully functional operational business model for a more specialized, industry specific application system.

The invention is defined in the independent claims to which reference should now be made. Preferred features are set out in the sub-claims.

Embodiments of the present invention provides for converting and uploading industry specific data from an existing ("legacy") database, e.g. a more general, tailored information system, into a new, industry specific information database.

In an embodiment, the present invention provides methods for providing accessibility to a database by a class of personnel for a predetermined industry. Once the predetermined industry is selected, legacy data relevant to the industry is retrieved, such as from a non-object oriented database first source, and the retrieved data is processed to be suitable for incorporation in a transitional database. One or more predetermined rules is applied to the processed data to ensure compatibility of the processed data with the requirements of the transitional database. The processed data is then incorporated into the transitional database if the data is determined to be compliant with the rules and the transitional database data is communicated to an industry specific information database, e.g. an object-oriented database.

The transitional database further comprises a predetermined initial structure and predetermined data. For a predetermined industry comprising healthcare information systems, e.g. for use in clinical care delivery, the data is typically related to patients.

In an alternative embodiment, the present invention further provides an intuitive, complete, and structured way for migrating information from an older information system towards a new, more industry specific system. A user interface is provided in an embodiment of the invention wherein at least one display window is generated to support merging data elements held in first and second data repositories (for example, a legacy data base and a starter model) into a composite data repository. The display window contains a representation of items derived from the first repository presented horizontally adjacent to items derived from the second repository. This permits side by side comparison. Once displayed, the user interface supports merging the selected individual data items into a composite repository in response to user command. For example, selection icons may be used to permit user selection of individual items from the first and second repositories for inclusion in the composite repository.

The skilled person will appreciate that features from the alternative embodiments can be combined where not evidently incompatible.

The scope of protection is not limited by the summary of an exemplary embodiment set out above, but is only limited by the claims.

These and other features, aspects, and advantages of the present invention will become more fully apparent from the following description, appended claims, and accompanying drawings in which:
**Fig. 1** is a schematic overview of an embodiment of the present invention;
**Fig. 2** is a schematic of representative tables;
**Fig. 3** is an exemplary display of a user interface display;
**Fig. 4** is an exemplary display of a user interface display;
**Fig. 5** is an exemplary display of a user interface display;
**Fig. 6** is a flowchart of an exemplary embodiment; and
**Fig. 7** is an exemplary display of a user interface display.

In general, throughout this description, if an item is described as implemented in software, it can equally well be implemented as hardware. It is also understood that "data," as used herein, is either singular or plural as the context requires.

The present invention may be used to minimize customer implementation time required to transition from an existing software application system and use its legacy data in a new system. As used herein, "legacy" data is data from a prior or currently existing system. The present invention may further be used to enable a customer to initiate operation of a fully functional operational business model for a more specialized, industry specific application system. The present invention may be used to minimize the need for specific customer adaptation of an industry specific system, as this type of customization significantly prolongs the time interval between delivery and start-up.

In a preferred embodiment, the present invention provides tools to load reference files with data from a universal data set and uses conversion routines to convert existing data into a format for a specific industry. In a preferred embodiment, a system according to the present invention is supported under a WINDOWS® NT® environment using a structured system query language ("SQL") database such as MICROSOFT® SQL Server. However, the present invention is not limited to a specific operating environment or database application system. Further, multiple users may access, update, and import data simultaneously for a single or multi-entity environment, e.g. including local area network (LAN) or wide area network (WAN) based systems. Moreover, embodiments of the present invention may provide administrative access such as for assigning users and tasks.

Although a healthcare system is used in this detailed description of a preferred embodiment to describe an exemplary embodiment, the present invention is not limited to healthcare systems.

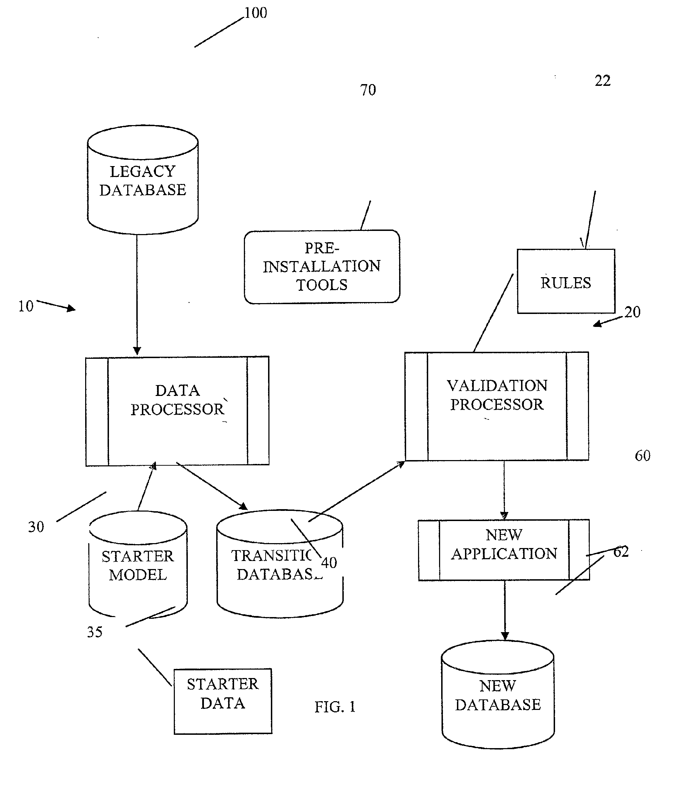

Referring now to **Fig. 1**, a schematic of an exemplary embodiment of the present invention for use in providing an industry specific information database accessible by personnel for use in that industry, a system according to the present embodiment comprises data processor 10, validation processor 20, and starter model 30. As described more fully herein below, starter model 30 comprises transition database 40 and software applications to access and manipulate components of starter model 30.

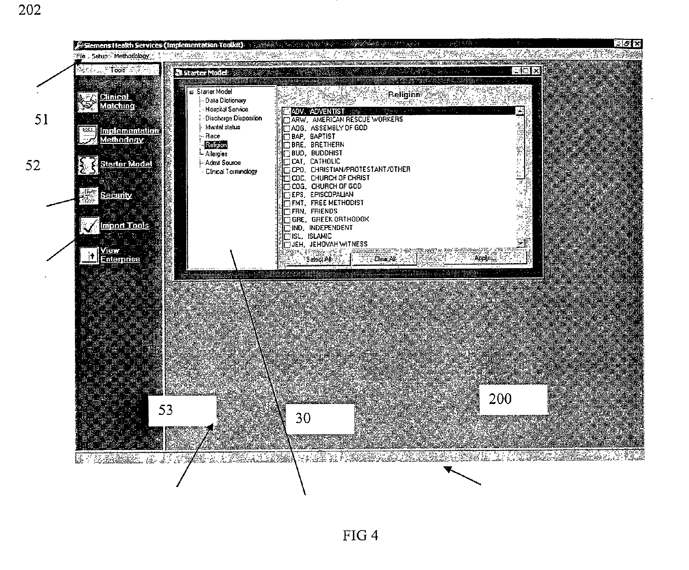

Data processor 10 may be a dedicated computer or a software process executing in one or more computers. Data processor 10 may further comprise display 12 (not shown in the figures) on which user interface 200 (**Fig. 4**) may be displayed, as well as input device 14 (not shown in the figures). As will be understood to those of ordinary skill in the computer arts, input device 14 may be a keyboard, mouse, pointing device, biometric device, or the like, or combinations thereof.

Database 100 comprises preexisting, legacy data generated by an existing system which reflect a targeted industry specific environment, e.g. the healthcare industry. In a preferred embodiment, a predefined flat file structure may be supplied for each type of data that resides in the legacy database, e.g. in database 100. This flat file structure may be used during an import process, but the end user can also import the data through a variety of other forms, e.g. a MICROSOFT® EXCEL® spreadsheet, an hypertext markup language (HTML) or extensible hypertext markup language (XML) file, a file formatted using a standard such as health level seven, or the like. Accordingly, as used herein, database 100 may be in a relational database format, a flat file format, a spreadsheet format, a comma delimited format, a hypertext format, an industry standardized format, or the like, or a combination thereof.

Data processor 10 is used to retrieve legacy data from database 100 and process the legacy data to be suitable for incorporation in transitional database 40. In an exemplary healthcare embodiment, the legacy data comprise patient related data.

Validation processor 20 may be used for applying predetermined rules 22 to determine whether the processed data is compatible with a required structure, e.g. the particular record structure of transitional database 40. For example, rule 22 may comprise a validation of the presence of address information for the patient because if a patient's received demographic data is all that is available, e.g. statistical information such as a patient's age or sex or income, the system may not have sufficient information to create an address record for the patient. Rules 22 may further include validation that sufficient information exists to satisfy a specific record's fields such as data type or data content, validation that field data is properly formatted, validation that field data is within a predetermined range of permissible values, or the like, or a combination thereof. Validation processor 20 may use predetermined rules 22 and notify the system that required data is missing. As used herein, validation processor 20 may be a computer or software process in a computer that is separate from data processor 10 or a separate process executing with data processor 10.

In a preferred embodiment, starter model 30 further comprises software applications to access and manipulate starter model 30, and possibly transition database 40, and pre-existing data, e.g. database 100, for transitioning from a first application system to a fully developed new application system. Starter model 30 may be used to process industry-standard information to support business process workflow.

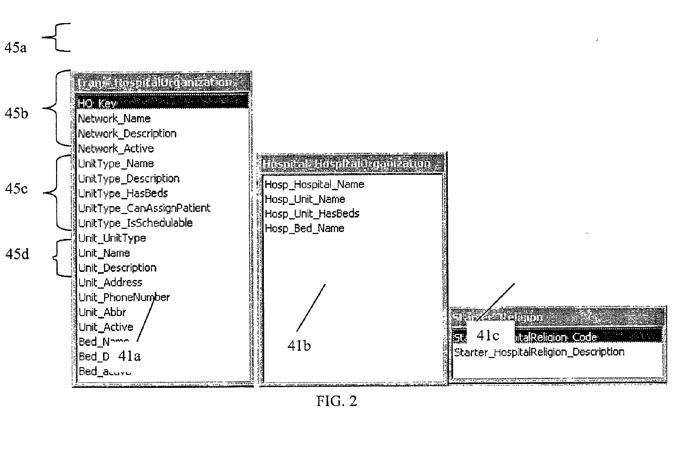

Additionally, starter model 30 comprises starter (or template) data 35, e.g. industry specific data, and may further comprise tool setup data. For example, a client may load starter model 30. Objects, e.g. an object belonging to a class such as object classes Network 45a, Unit Type 45b, Unit 45c, or Bed 45d, would be defined by a new database, e.g. healthcare information database 60. Transitional database 40 therefore needs to have access to definitions of elements of objects that are required to create the object with a new database, e.g. healthcare information database 60. Starter data further comprises a predetermined amount of data as required for required classes 45 (shown in **Fig. 2** as classes 45a, 45b, and 45c) such as those that may be present in transitional database 40. For example, the predetermined amount of data may include initial descriptions of religions, initial descriptions of bed types, and the like, or combinations thereof.

For example, starter model 30 for accident codes may comprise elements as shown in Table 1:

**TABLE 1**

| Abbreviation | Name | Description |
|---|---|---|
| A | Automobile | Automobile make and model |
| H | Home | Home address |
| O | Other | Miscellaneous information |
| S | School | School information |
| W | Work | Work information |

By way of further example, starter model 30 for allergy codes may comprise elements as shown in Table 2:

**TABLE 2**

| Abbreviation | Name | Description |
|---|---|---|
| A | Drug allergy | Drug allergy |
| FA | Food allergy | Food allergy |
| MA | Miscellaneous allergy | Miscellaneous allergy |
| MC | Miscellaneous contraindication | Miscellaneous contraindication |
| EA | Environmental Allergy | Environmental Allergy |
| AA | Animal Allergy | Animal Allergy |
| PA | Plant Allergy | Plant Allergy |
| LA | Pollen Allergy | Pollen Allergy |

Transitional database 40 has a predetermined record structure and is used to incorporate the processed legacy data which is determined to be integratable into transitional database 40, e.g. by storing the integratable information for subsequent transfer to a new application database such as healthcare information database 60. For example, a record in transitional database 40 may comprise fields for patient name, billing address, insurance, person to notify, allergies, medical condition, and religion. A field in a record in the legacy data may not have a counterpart in transitional database 40 and would therefore not be integrated into transitional database 40, e.g. a field containing data relevant to a no longer used option. Transitional database 40 may further comprise user specific data and starter database elements.

Transitional database 40 may comprise transitional tables 41 (**Fig. 2**) used for incorporating both user specific data and starter database elements. Further, transitional tables 41 of transitional database 40 may be used to populate a new database, e.g. healthcare information database 60. Each transitional table 41 may comprise one or more object class 45 (such as 45a, 45b, and 45c in **Fig. 2**) to be used within healthcare information database 60. This allows the population process to take full advantage of predetermined common objects or classes of objects in forming transitional database 40, a new database such as healthcare information database 60, or both.

Referring additionally to **Fig. 2,** in an exemplary healthcare embodiment, tables, referred to generally herein as "41" and shown in the figure as tables 41a, 41b, and 41c, may further use naming conventions to aid in the conversion. For example, Trans_HospitalOrganization transitional table 41a is shown having object classes Network 45a, Unit Type 45b, Unit 45c, and Bed 45d. These classes 45 are used to create an exemplary healthcare information database 60. As shown in **Fig. 2**, for example, in an exemplary embodiment table 41 with starter data, e.g. table 41c, may precede field names with "Starter_" then append the type of data, e.g. a "HospitalReligionCode" data field would be named "Starter_HospitalReligionCode". In a similar manner, table 41 that stores hospital imported data, e.g. 41b, may precede their names with "Hospital_," e.g. "Hospital_HosptialOrganization".

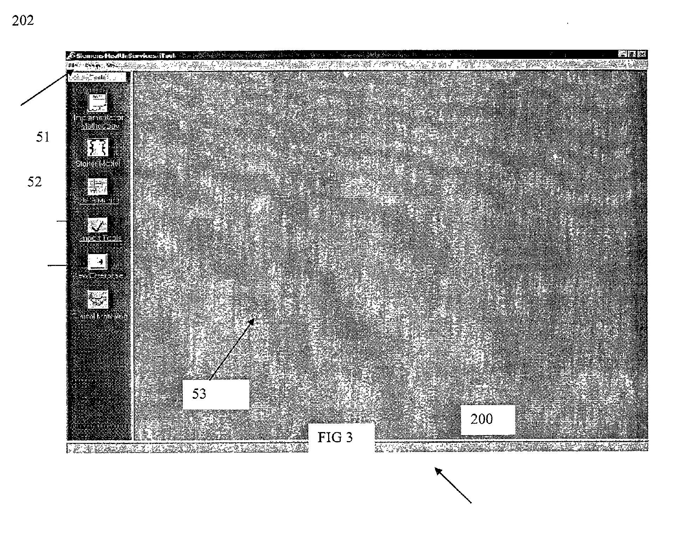

Referring now to **Fig. 3**, in a preferred embodiment, transitional database 40 is accessible from user interface 200 such as shown in **Fig. 3** through **Fig. 5.** User interface 200 may comprise user selectable image elements, such as import image element 51 which can be invoked to begin functions that allow importing the legacy data, and export image element 52, which can be invoked to begin functions to export data in a predetermined format according to industry specific data required by the new application. Import image element 51 and export image element 52 may comprise functionality to implement their respective functions in an interactive mode, a batch mode, or a combination thereof, e.g. interactive menus, scripts, and the like.

Referring additionally to **Fig. 4,** for interactive access, user interface 200 may provide an end user with an ability to import enterprise specific legacy data, e.g. data in database 100, in numerous formats which are to be supported in a new system. The import enterprise specific legacy data are therefore integratable by using the data, once imported, during a merge process to merge the integratable legacy data with starter data 35 (**Fig. 1**) supplied in starter model 30. During this merge process, one or more executable procedures may be invoked to scan a predetermined portion of source data to ensure there are not any duplicates, e.g. using validation processor 20 (**Fig. 1**). Once verified, one or more executable procedures may then commit the data to appropriate tables 41 (**Fig. 2**). Tables 41 containing the merged data may further be marked as combined. Starter data 35 may be pre-populated to reflect data and data formats common or otherwise related to a specific industry.

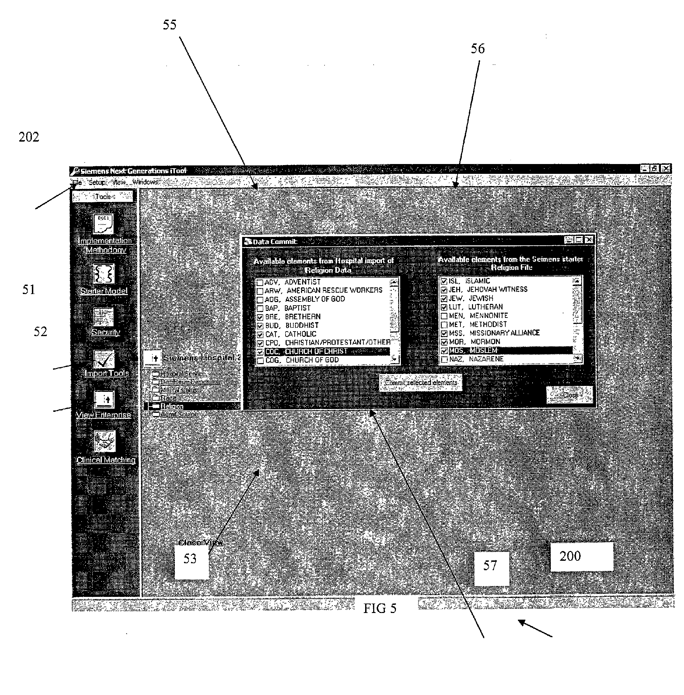

Referring now to **Fig. 5,** a user may elect to use user interface 200 to simultaneously display a representation of two or more datasets, e.g. dataset 55 from database 100 and dataset 56 from starter model 30 (**Fig. 1**). The user may be allowed to choose which elements from either side (database 100 or starter model 30) are to be used to populate transitional table 41.

When the end user commits data to transitional database 40 (**Fig. 1**), the system may then apply one or more predetermined rules 22 (**Fig. 1**) against the dataset reflected in transitional database 40 to further determine data integrity. Validated data may then be committed to transitional tables 41 (**Fig. 2**) of transitional database 40 in the record format required by these transitional tables 41. In a preferred embodiment, this commitment process is driven by a user's task list.

In the operation of an exemplary embodiment, use of the present invention is task driven. A user is presented with tasks that need to be completed in order to implement a new information system and its associated modules. Additionally, one or more data-gathering tools may be provided to enable a user to define, extract, import, and cleanse, e.g. validate, data to be used in an electronic database implementation prior to delivery of a new software application.

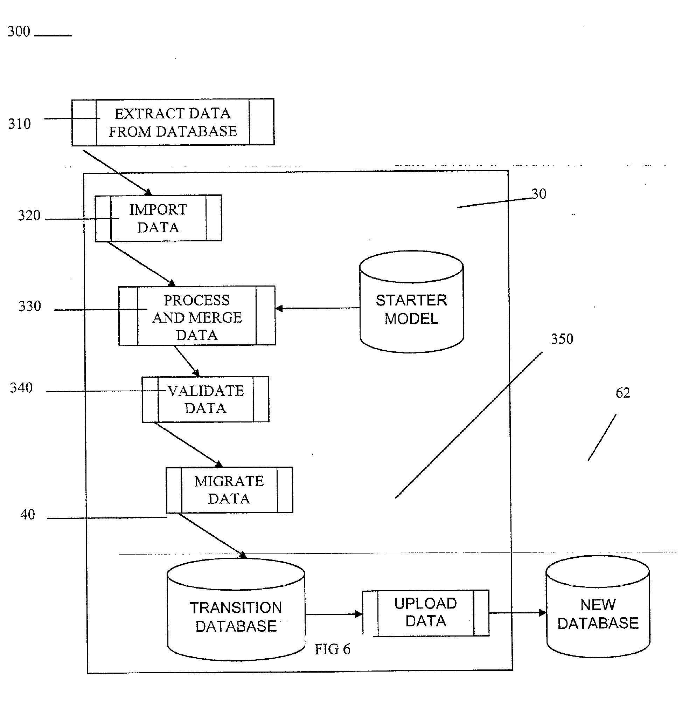

Referring now to **Fig. 6**, an end user may first initiate the present invention such as at a workstation. By way of example and not limitation, a user may invoke software embodying the present invention's method from a network workstation that provides access to an implementation desktop menu. The user may log into the present invention using numerous equivalent methods as will be familiar to those of ordinary skill in the software arts, including using a login ID. If used, a user's login ID may be used to track the status of implementation tasks.

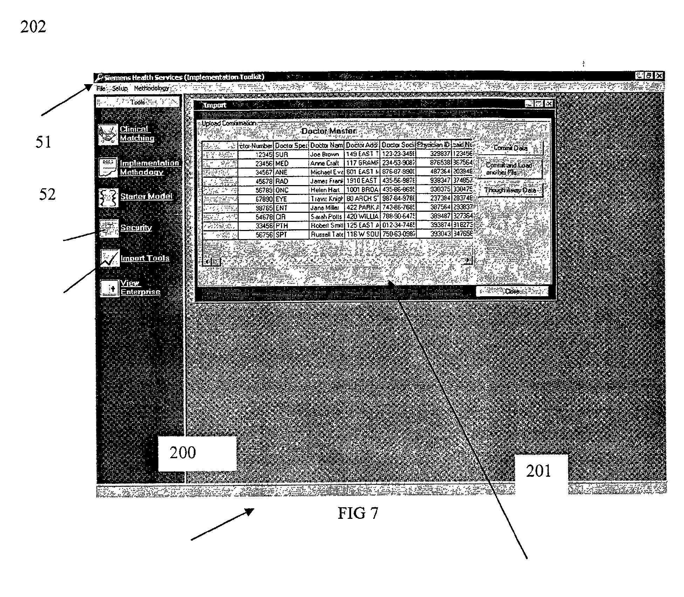

Once logged in, the user may initiate generation of at least one display window 53 (**Fig. 3**) such as at display 12 (not shown in the figures). Once user window 53 is displayed, the user may select a new task or select and complete a previously started task, e.g. from form 201 (**Fig. 7**), menu 202 (**Fig. 7**), or the like. When a task is selected, the steps to complete the selected task may be displayed for the end user. For example, database 100 may be queried, e.g. programmatically, for a list of tasks assigned to the end user. The end user may then be presented with a list of tasks and their associated status as a result of the query. One or more menus 202 may then allow the user assigned to a specific task to select a desired task from the end user's worklist. As used herein, a worklist is a listing of work items assigned to a user or other task performer based on a specific assignment strategy.

Data is typically extracted, step 300, from an existing database, e.g. legacy data is accessed from database 100 (**Fig. 1**) and received into data processor 10 (**Fig. 1**). For example, a user can be prompted for and select a desired database 100 from which data may be imported. In an exemplary healthcare application, patient related data is accessed and retrieved from a first source, e.g. database 100 (**Fig. 1**), which can be either an object-oriented database or a non-object oriented database or a combination thereof, where the received patient related data may be obtained by migrating data from a relational database, a flat file, a spreadsheet, an HTML file, an XML file, a file formatted using the health level seven format, e.g. HL7-A28, or the like, or a combination thereof.

Extracted data is then imported, step 310, into one or more tables 41 (**Fig. 2**) and modified, 320, using data from starter model 30, e.g. starter data 35 (**Fig. 1**). For example, a record containing a date data field may have the date field changed from a two digit year format into a four digit year format. The step of processing received patient related data may further include the step of including data from a template database together with the received patient related data. Processed patient related data may be further examined to determine whether the processed patient related data are textually valid, numerically valid, or the like, or a combination thereof.

In an exemplary healthcare embodiment, the received patient related data is processed to be suitable for incorporation in transitional database 40. Transitional database 40 will further comprise at least one transitional table 51 (not shown in the figures) which may further comprise at least one object class 45 (**Fig. 2**). Accordingly, processing the received patient related data, such as at step 320, may further comprise re-formatting received patient related data such as to include particular data required by an object associated with object class 45. Processing the received patient related data may further comprise parsing received patient related data to identify data elements for inclusion in predetermined data fields in the particular record structure of transitional database 40, deriving data elements from received patient related data for inclusion in predetermined data fields in the particular record structure of transitional database 40, omitting data elements of received patient related data from the particular record structure of the transitional database 40, or the like, or a combination thereof.

Processing the received patient related data may further comprise including data from a template database, e.g. starter model 30, together with received patient related data. Therefore, in an exemplary healthcare application, a user who wishes to convert a current application system's data to data accessible by a new application system, such as one to be accessible by healthcare personnel for use in clinical care delivery, accordingly populates transitional database 40 with predetermined starter data 35 representative of healthcare data processing requirements. These starter data 35 may comprise recognized or de facto industry standards, e.g. for a specific targeted use such as healthcare.

Referring back to **Fig. 5,** user interface 200 may provide one or more regions on display 12 (not shown in the figures) indicating items derived from dataset 55 representing data in database 100 as well as dataset 56 presenting data from starter model 30 The two databases, 55 and 56, may be presented horizontally adjacent, permitting side by side actions such as comparison and selection. The items derived from the first and second datasets 55,56 may comprise data elements held in the first and second datasets 55,56, identifiers indicating categories of data elements in datasets 55,56, identifiers indicating data fields of records in datasets 55,56, or combinations thereof. Selection icons, e.g. checkboxes, may exist to permit user selection of individual items of the items derived from the datasets 55,56 for inclusion in a composite repository.

User interface 200 may also support merging data elements held in datasets 55,56 into a composite repository. For example, the user may initiate merger of the selected individual data items into a composite repository in response to user command such as by selecting an icon, a keyboard action, a mouse action, or the like, or a combination thereof.

Referring back to **Fig. 6**, merged data is validated, step 330, and then migrated, step 340, into transitional database 40. In a preferred embodiment, a task may not be marked as completed until data has passed validation checks, e.g. data integrity validation.

Once all the steps for a task are complete, the user or the system may then mark the task complete. If no data were involved, the user or system may mark the task completed. The system then stores predetermined parameters for the completed task in a logfile, e.g. parameters such as date, time, and user's sign-on ID. If data were involved, the system may run an internal data integrity check to verify that the targeted application system can use the data. Such an internal data integrity check may involve field checking, e.g. data type matching, boundary validation such as data being within an appropriate range, duplications, key values, and the like, or combinations thereof.

If the data passes the check, the task may be marked complete. If the data is found to have errors, e.g. the data do not comply with the requisite data type, the task is left uncompleted and the user notified such as by the display of an error warning in display window 53 (**Fig. 3**). Users can then either correct the data manually, such as exemplified in **Fig. 7**, or automatically under program control according to predetermined data validation rules.

When valid processed data exist, the processed data is merged with starter data 35 in transitional tables 41, i.e. validated data may then be committed to a predetermined transitional table 41 of transitional database 40 in a desired record format for that predetermined transitional table 41.

Merged, processed data may be examined under program control and/or manually to determine whether the processed data is valid, e.g. textually valid, numerically valid, valid for a field type, or a combination thereof. Additionally, the traditional database data may be reviewed by users who may manually modify traditional database 100, e.g. users of new application system 60 such as healthcare personnel.

Referring back to **Fig. 6**, after processing, one or more predetermined rules 22 (**Fig. 1**) such as field validation rules or data formatting rules may be applied to the processed data to determine whether the processed data is compatible with transitional database 40.

Migrated data may then be uploaded, step 350, into a new application with its new database, e.g. database 62. For example, in the exemplary healthcare embodiment, the processed patient related data determined to be compatible with a new healthcare system are incorporated into transitional database 40 and transitional database data communicated to a user healthcare information database, e.g. 62. An example of such a database system is the SOARIAN™ Health Information Solution system manufactured by SMS Enterprises, Inc. of Delaware. Communicating data content may include sequentially ordering data elements of the content for communication by ordering the data content to be compatible with a desired hierarchical record structure. For object oriented targeted systems, communicating data content may include creating linked object elements for inclusion in a desired record structure of user healthcare database 62 wherein the linked objects reflect the desired record structure.

After the task is complete, the user can proceed to the next task, research any issues reported by the system, or logoff to complete the tasks at a later date.

Referring back to **Fig. 1,** in a currently envisioned embodiment, pre-installation tools 70, e.g. a workbook and system tools, may be used to enable a customer to review and modify data in starter model 30 and load data from existing systems, e.g. database 100.

These pre-installation tools 70 may further comprise a documented process flow that includes system settings, predetermined rules, and other installation model assumptions to allow a customer to appraise and identify any required adaptation. Specifically, for a healthcare embodiment information may be gathered identifying input file formats, e.g., laboratory terms, radiology terms, drug catalogs, and the like, that are to be processed as well as identifying other requirements that may be implemented in a starter model or after installation of a starter model, e.g., user security requirements, and the like.

It will be understood that various changes in the details, materials, and arrangements of the parts which have been described and illustrated above in order to explain the nature of this invention may be made by those skilled in the art without departing from the scope of the invention as recited in the following claims.

## Claims

1. A method for providing accessibility to a healthcare information database by healthcare personnel for use in clinical care delivery, comprising the steps of:
a. retrieving patient related data;
b. processing the retrieved patient related data to be suitable for incorporation in a transitional database, the transitional database including:
i. a predetermined initial structure; and
ii. predetermined data;
c. applying a predetermined rule to the processed patient related data to ensure compatibility of the processed patient related data with the transitional database;
d. incorporating the processed patient related data into the transitional database in response to the determination; and
e. communicating transitional database data to a user healthcare information database.

2. A method according to claim 1, wherein:
the transitional database further includes at least one transitional table, the at least one transitional table further comprising at least one object class.

3. The method according to claim 2, wherein:
the step of processing the received patient related data further comprises re-formatting the received patient related data to include particular data required by an object associated with the at least one object class.

4. A method according to any of the preceding claims, wherein:
the step of processing the received patient related data comprises at least one of (i) parsing the received patient related data to identify data elements for inclusion in predetermined data fields in the particular record structure of the transitional database, (ii) deriving data elements from the received patient related data for inclusion in predetermined data fields in the particular record structure of the transitional database, or (iii) omitting data elements of the received patient related data from the particular record structure of the transitional database.

5. A method according to any of the preceding claims, wherein:
the step of processing the received patient related data includes the further step of including data from a template database together with the received patient related data.

6. A method according to any of the preceding claims, further including the step of:
examining the processed patient related data to determine whether the processed patient related data is at least one of (i) textually valid or (ii) numerically valid.

7. A method according to any of the preceding claims, wherein:
the predetermined rule is at least one of (i) validation that sufficient information exists to satisfy a record's fields, (ii) validation that field data is properly formatted, or (iii) validation that field data is within a predetermined range of permissible values.

8. A method according to any of the preceding claims, wherein:
the step of communicating data content of the transitional database to a user healthcare information database includes the further step of sequentially ordering data elements of the content for communication by ordering the data content to be compatible with a desired hierarchical record structure.

9. The method according to any of the preceding claims, wherein step (a) further comprises:
receiving patient related data by migrating data from at least one of (i) a relational database, (ii) a flat file, (iii) a spreadsheet, (iv) a hypertext file, (v) an extensible markup language (XML) formatted file, or (vi) an health level seven (HL7-A28) formatted file.

10. A method for providing a user interface supporting merger of patient related data elements derived from a first repository and record data elements derived from a second repository, comprising the steps of:
f. initiating generation of at least one display window supporting merging a data element held in the first repository and a data element held in the second repository into a composite repository, the at least one display window including:
i. an element derived from the first repository being presented horizontally adjacent to an element derived from the second repository permitting side by side comparison; and
ii. a selection icon permitting user selection of an individual element derived from the first repository and an individual element derived from the second repository for inclusion in the composite repository; and
g. initiating merger of the selected individual data element into a composite repository in response to user command.

11. A method according to claim 10, wherein:
the element derived from the first repository and the element derived from the second repository are at least one of (i) data elements held in either of the first or second repositories, (ii) identifiers indicating a category of data elements in the first and second repositories, or (iii) identifiers indicating a data field of a record in either of the first or second repositories.
